# EUROPEAN PATENT APPLICATION

(11) **EP 4 375 356 A1**
(43) Date of publication of application: **29.05.2024**
(21) Application number: 22845824.6
(22) Date of filing: 13.07.2022
(51) Int. Cl.: C12M 1/00, C12N 5/07, C12N 5/071, C12N 5/10, C12N 1/00, C12N 15/87, C12Q 1/02

(54) **MEANS FOR CONTROLLING INTRACELLULAR REACTION UTILIZING NEEDLE-SHAPED BODY**

(30) Priority: 20.07.2021 JP 2021119463; 20.07.2021 JP 2021119466
(71) Applicant: Toyo Seikan Group Holdings, Ltd., Shinagawa-ku Tokyo 141-8627 (JP)
(72) Inventor: TOTANI, Takahiko, Yokohama-shi, Kanagawa 240-0062 (JP)
(74) Representative: Jones, Nicholas Andrew
(86) International application number: PCT/JP2022/027506
(87) International publication number: WO 2023/002890

(57) **Abstract**

An object of the present invention is to provide a novel technique or method for controlling intracellular reaction that allows introduction of a specific factor into a large number of cells with simple and efficient operations, and the present invention provides a method for controlling intracellular reaction, the method including the steps of: bringing a needle-shaped body having an intracellularly introducible factor immobilized thereon into contact with a cell and inserting a part of the needle-shaped body into the cell to introduce the intracellularly introducible factor into the cell; and withdrawing the part of the needle-shaped body from the cell.

## Description

### Technical Field

The present invention relates to a method for introducing an intracellularly introducible factor immobilized on a needle-shaped body into a large number of cells in a simple and efficient manner, and a means for controlling intracellular reaction utilizing the method.

In addition, the present invention relates to a needle-shaped particle having a binding substance capable of binding to an intracellular molecule immobilized thereon for use in controlling intracellular reaction, a cell processing composite substrate including the needle-shaped particle, and a method for controlling intracellular reaction utilizing any of them.

### Background Art

Various techniques and methods for controlling intracellular reaction have been developed and reported today. However, most of them involve use of gene recombination techniques and genome editing techniques, which generally need cumbersome operations and huge cost and the like and impose heavy burdens on operators. In addition, concerns may arise, such as the possibility that a factor that has been intracellularly introduced through the process of a gene recombination technique or genome editing technique itself causes certain intracellular reaction and the possibility that such a factor remains in cells. Therefore, a novel technique or method for controlling intracellular reaction is strongly demanded in the art still now.

Patent Literature 1 discloses a needle-shaped material for cell insertion with an antibody against an intracellular or intercellular protein antigen immobilized thereon, and states that the needle-shaped material positioned to a cell and inserted into the cell through up-and-down movement allows quantification, evaluation, and so on for an intracellular or intercellular protein in the cell kept alive.

Patent Literatures 2 and 3 disclose a cell insertion member including a large number of nanoneedles arranged on a support, wherein the cell insertion member is formed by means of photolithography and dry etching and wet etching methods, and state that a large number of nanoneedles, on each of which, for example, a gene or a substance involved in gene expression or a substance that binds to a marker substance such as an antibody has been immobilized in advance, allow, when simultaneously inserted into a large number of cells arranged on a substrate, analysis of gene expression state in the large number of cells being still alive and allow target cells to be selectively pulled up.

However, those needle-shaped material and nanoneedles may require cumbersome operations for positioning or up-and-down movement of them in inserting into cells or the like and impose heavy burdens on operators, and a substance to be immobilized on the needle-shaped material or nanoneedle such as an antibody may bind also to, for example, a part (on the support) other than the needle-shaped material or nanoneedle, resulting in the loss (waste) of the substance.

### Citation List

### Patent Literature

Patent Literature 1: Japanese Patent Laid-Open No. 2006-246731
Patent Literature 2: Japanese Patent Laid-Open No. 2013-183706
Patent Literature 3: Japanese Patent Laid-Open No. 2006-166884

### Summary of Invention

### Technical Problem

An object of the present invention is to provide a novel technique or method for controlling intracellular reaction that allows introduction of a specific factor into a large number of cells with simple and efficient operations.

Another object of the present invention is to provide a novel technique or method for controlling intracellular reaction that allows processing of a large number of cells with simple operations without causing a factor that has been introduced to remain in cells after the operations.

### Solution to Problem

The present inventors diligently studied to solve the problems, and as a result found that a needle-shaped part of a needle-shaped body was more efficiently inserted into a cell when the needle-shaped body was brought into contact with a cell having a weight bound thereto than when the needle-shaped body was brought into contact with a cell not having a weight bound thereto; and that an intracellularly introducible factor can be introduced into a large number of cells in a simple and efficient manner by immobilizing the introducible factor on a needle-shaped body and bringing the resultant into contact with a cell having a weight bound thereto; thus, intracellular reaction can be, for example, controlled or analyzed.

The present invention is based on those novel findings, and includes the followings.
[1] A method for producing a modified cell, the method including the steps of:
   bringing a needle-shaped body having an intracellularly introducible factor immobilized thereon into contact with a cell having a weight bound thereto and inserting a part of the needle-shaped body into the cell to introduce the intracellularly introducible factor into the cell; and
   withdrawing the part of the needle-shaped body from the cell.
[2] The method according to [1], wherein the weight is made of one or more substances selected from the group consisting of resins, metals, magnetic substances, and any combination of them.
[3] The method according to [1] or [2], wherein the weight is in a bead-like form.
[4] The method according to any of [1] to [3], wherein the step of inserting a part of the needle-shaped body into the cell and/or the step of withdrawing the part of the needle-shaped body from the cell are/is performed by means of one or more external forces selected from the group consisting of centrifugal force, magnetic force, water flowing, water pressure, and electrostatic interaction.
[5] The method according to any of [1] to [4], wherein the intracellularly introducible factor is a binding substance capable of binding to an intracellular molecule, and the method includes withdrawing the needle-shaped body from the cell to withdraw an intracellular molecule bound to the binding substance in the cell together with the needle-shaped body from the cell.
[6] The method according to [5], wherein the binding substance is a nucleic acid, a protein, a peptide, or a low-molecular-weight compound.
[7] The method according to [5] or [6], wherein the binding substance is an antibody or a fragment thereof.
[8] The method according to any of [1] to [4], wherein the intracellularly introducible factor is a bioactive substance, and the method includes withdrawing the needle-shaped body from the cell to allow the intracellularly introducible factor to remain in the cell.
[9] The method according to [8], wherein the bioactive substance is one or more substances selected from the group consisting of nucleic acids, peptides, proteins, saccharides, polysaccharides, fatty acids, cholesterols, lipids, signal transducers, ligand substances, hormonal substances, cytokines, ions, metal particles, magnetic microparticles, inorganic compounds, quantum dots, organic compounds, and drugs.
[10] The method according to [8] or [9], wherein a bond between the bioactive substance and the needle-shaped body is an intracellularly separable bond.
[11] The method according to [10], wherein the bond between the intracellularly introducible factor and the needle-shaped body is at least one selected from the group consisting of electrostatic bonds, bonds formed through hydrophobic interaction, chelating bonds, intracellularly cleavable covalent bonds, bonds via a photocleavable linker, and bonds via an enzymatically cleavable linker.
[12] The method according to any of [1] to [11], wherein the needle-shaped body is a needle-shaped particle.
[13] The method according to [12], wherein a needle-shaped part of the needle-shaped particle has a length of 1 to 50 µm.
[14] The method according to [12] or [13], wherein the needle-shaped particle is made of zinc oxide.
[15] The method according to any of [1] to [14], wherein the needle-shaped body is in an immobilized form on a substrate.
[16] The method according to [15], wherein the substrate includes a binder on a surface of the substrate, and a part of the needle-shaped body is immobilized on the substrate via the binder.
[17] The method according to [16], wherein the binder consists of a protein-nonadsorptive material.
[18] The method according to [15], wherein the needle-shaped body is a needle-shaped body produced by using one or more selected from the group consisting of photolithography, dry etching, wet etching, and any combination of them.
[19] The method according to any of [1] to [18], wherein the substrate includes a fine uneven structure capable of accommodating a cell in a face of the substrate with the needle-shaped body.
[20] A method for analyzing a function of an intracellularly introducible factor, the method including the step of: analyzing the state of a modified cell produced by using the method according to any of [1] to [19].
[21] The method according to [20], wherein, in the step of analyzing the state of a modified cell, analysis is performed for the cell on one or more selected from the group consisting of the growth rate, the viability, metabolism, quantification of reactive oxygen species, and gene expression.

In addition, the present inventors diligently studied to solve the problems, and as a result found that use of a needle-shaped particle having a binding substance capable of binding to an intracellular molecule immobilized thereon, and use of a cell processing composite substrate including the needle-shaped particle allow the targeted intracellular molecule to be withdrawn by inserting a needle-shaped part into a cell, which allows processing of a large number of cells with simple operations, and intracellular reaction can be, for example, controlled or analyzed without allowing a factor that has been introduced to remain in cells after the operations.

The present invention is based on those novel findings, and includes the followings.
<1> A needle-shaped particle having a binding substance capable of binding to an intracellular molecule immobilized thereon.
<2> The needle-shaped particle according to <1>, wherein a needle-shaped part of the needle-shaped particle has a length of 1 to 50 µm.
<3> The needle-shaped particle according to <1> or <2>, wherein the needle-shaped particle is made of zinc oxide.
<4> The needle-shaped particle according to any of <1> to <3>, wherein the binding substance is a nucleic acid, a protein, a peptide, or a low-molecular-weight compound.
<5> The needle-shaped particle according to <4>, wherein the binding substance is an antibody or a fragment thereof.
<6> A cell processing composite substrate including a needle-shaped particle immobilized on a substrate.
<7> The cell processing composite substrate according to <6>, wherein a needle-shaped part of the needle-shaped particle has a length of 1 to 50 µm.
<8> The cell processing composite substrate according to <6> or <7>, wherein the needle-shaped particle is made of zinc oxide.
<9> The cell processing composite substrate according to any of <6> to <8>, wherein the substrate consists of a resin.
<10> The cell processing composite substrate according to any of <6> to <9>, wherein the substrate comprises a binder on a surface of the substrate, and a part of the needle-shaped particle is immobilized on the substrate via the binder.
<11> The cell processing composite substrate according to <10>, wherein the binder consists of a protein-nonadsorptive material.
<12> The cell processing composite substrate according to any of <6> to <11>, wherein a fine uneven structure capable of accommodating a cell is formed in a face of the substrate with the needle-shaped particle immobilized thereon.
<13> The cell processing composite substrate according to any of <6> to <12>, wherein a binding substance capable of binding to an intracellular molecule is immobilized on the needle-shaped particle.
<14> The cell processing composite substrate according to <13>, wherein the binding substance is a nucleic acid, a protein, a peptide, or a low-molecular-weight compound.
<15> The cell processing composite substrate according to <14>, wherein the binding substance is an antibody or a fragment thereof.
<16> A method for producing a modified cell, wherein a function of an intracellular molecule is removed or reduced, the method including the steps of:
   inserting a part of the needle-shaped particle according to any of <1> to <5> or the needle-shaped particle in the cell processing composite substrate according to any of <13> to <15> into a cell;
   allowing the intracellular molecule and the binding substance immobilized on the needle-shaped particle to bind together; and
   withdrawing the needle-shaped particle together with the intracellular molecule bound thereto via the binding substance from the cell.
<17> The method according to <16>, wherein the modified cell is used for analyzing the function of the intracellular molecule.
<18> The method according to <16> or <17>, wherein the step of inserting a part of the needle-shaped particle into a cell and/or the step of withdrawing the needle-shaped particle from the cell are/is performed by means of one or more external forces selected from the group consisting of centrifugal force, magnetic force, water flowing, water pressure, and electrostatic interaction.
<19> The method according to any of <16> to <18>, wherein a weight is bound to the cell.
<20> A method for analyzing a function of an intracellular molecule, the method including the steps of:
   inserting a part of the needle-shaped particle according to any of <1> to <5> or the needle-shaped particle in the cell processing composite substrate according to any of <13> to <15> into a cell;
   allowing the intracellular molecule and the binding substance immobilized on the needle-shaped particle to bind together;
   withdrawing the needle-shaped particle together with the intracellular molecule bound thereto via the binding substance from the cell; and
   analyzing the state of the cell.
<21> The method according to <20>, wherein, in the step of analyzing the state of the cell, analysis is performed for the cell on one or more selected from the group consisting of the growth rate, the viability, metabolism, quantification of reactive oxygen species, and gene expression.
<22> The method according to <20> or <21>, wherein the step of inserting a part of the needle-shaped particle into a cell and/or the step of withdrawing the needle-shaped particle from the cell are/is performed by means of one or more external forces selected from the group consisting of centrifugal force, magnetic force, water flowing, water pressure, and electrostatic interaction.
<23> The method according to any of <20> to <22>, wherein a weight is bound to the cell.

The present specification includes the contents of specifications and others of Japanese Patent Application No. 2021-119463 and Japanese Patent Application No. 2021-119466, each filed on July 20, 2021, on which the priority of the present application is based.

All the publications, patents, and patent applications cited herein are directly incorporated herein by reference.

### Advantageous Effects of Invention

The present invention can provide a novel technique or method for controlling intracellular reaction that allows introduction of a specific factor into a large number of cells with simple and efficient operations.

In addition, the present invention can provide a novel technique or method for controlling intracellular reaction that allows processing of a large number of cells with simple operations without causing a factor that has been introduced to remain in cells after the operations.

### Brief Description of Drawings

[Figure 1] Figure 1 is a schematic diagram illustrating a method for producing a cell processing composite substrate including needle-shaped particles immobilized thereon.
[Figure 2] Figure 2 is a photograph for observation of needle-shaped particles (Pana-Tetra) immobilized on a cell processing composite substrate.
[Figure 3] Figure 3 is a series of graphs representing proportions of activated cells (CD25⁺ or CD137⁺) in PBMCs obtained by inserting and then evulsing needle-shaped parts of Pana-Tetra coated with an anti-Cbl antibody. Figures 3(A) and 3(B) respectively show results 1 day and 3 days after the initiation of culture.
[Figure 4] Figure 4 is a graph representing viable cell rates (i.e., efficiencies of insertion of needle parts of Pana-Tetra into cells) after cells were subjected to repeated processing with a cell processing composite substrate.
[Figure 5] Figure 5 is a graph representing proportions of differentiated cells (neurons indicated by a phenotype of CD56⁺) in MSCs (mesenchymal stem cells) obtained by inserting and then evulsing needle-shaped parts of Pana-Tetra coated with an anti-GSK3β antibody.

### Description of Embodiments

In the present invention, the term "needle-shaped body" refers to a fine structure including at least one needle-shaped part.

A "needle-shaped part" has a long and narrow shape that has a sufficiently small tip and width and, when inserted into a cell, causes less or almost no damage, preferably causes completely no damage to the cell. Each needle-shaped part may have any shape such as cylindrical, conical, tubular (e.g., prismatic), and pyramidal shapes (not limited thereto) as long as the shape satisfies the aforementioned requirement, and the tip may be sharp or not. For low invasiveness to cells and efficiency of insertion, cylindrical shape and conical shape are preferred. The length of each needle-shaped part is 0.5 to 100 µm, preferably 1 to 50 µm, more preferably 5 to 40 µm, and even more preferably 10 to 30 µm (e.g., 10 µm, 20 µm). The aspect ratio of each needle-shaped part can be about 5:1 to 50:1, and preferably 10:1 to 40:1, but is not limited thereto.

Each "needle-shaped body" consists of a material having low toxicity or almost no toxicity, preferably having completely no toxicity to cells, and examples of such materials include metal oxides (e.g., zinc oxide), inorganic substances (e.g., quartz, nickel oxide, silica, alumina, diamond, titania, zirconia), metals (e.g., gold, silver, copper, platinum, aluminum), metal crystals (e.g., tungsten, titanium, silicon crystals, zirconium), glass, resins (e.g., polyethylene, polypropylene, cyclic polyolefin, cyclic olefin copolymer, polyester, polystyrene, polymethyl acrylate, polylactic acid, polyether ether ketone, fluororesin), silicon nitride, and silicon (not limited thereto), and needle-shaped solid materials can be preferably used.

The form of each "needle-shaped body" can be any form that allows insertion of its needle-shaped part into cells and subsequent evulsion (also expressed as "withdrawal") without limitation, and, for example, each "needle-shaped body" can be in a form of a needle-shaped particle or in an immobilized form on a substrate.

The term "needle-shaped particle" in relation to the form of a "needle-shaped body" refers to a fine grain including at least one needle-shaped part. The shape of each needle-shaped particle is one in which one or more needle-shaped parts are arranged and/or bound in the center part, and can be, for example, rod-like, L-, V-, T-, Y-, or radial (e.g., tripodal, quadrupodal, tetrapodal) shape, but is not limited thereto. In the case that a needle-shaped particle includes a plurality of needle-shaped parts, the needle-shaped parts may have an identical shape and/or length in common, or have different shapes and/or lengths. Each needle-shaped particle may have a structure in which a plurality of needle-shaped parts is directly connecting to each other in the center part, or a structure in which one or more needle-shaped parts are connecting to a core member. The size of each needle-shaped particle may vary depending of the lengths and number of needle-shaped parts included therein, and can be such a size that the needle-shaped particle fits in a sphere having a radius of 0.5 µm to 100 µm.

Herein, numerical values for the sizes of the "needle-shaped body", the "needle-shaped particle", and the "needle-shaped part" are represented as average values.

In the present invention, each needle-shaped particle preferably consists of zinc oxide with a plurality of needle-shaped parts, and more preferably has a shape in which needle-shaped parts each consisting of a zinc oxide monocrystal of 10 to 20 µm in length are arranged like a tetrapod. For example, Pana-Tetra (R) WZ-0501 (manufactured by AMTEC Co., Ltd.) can be preferably used as such needle-shaped particles.

The phrase "an immobilized form on a substrate" means a substrate including a plurality of needle-shaped bodies (or needle-shaped parts) on a surface of the substrate. Hereinafter, a/the needle-shaped body or needle-shaped particle to be immobilized on a surface of a substrate is regarded as a plurality of needle-shaped bodies or needle-shaped particles, unless otherwise stated. The "substrate" may be any one that can immobilize the needle-shaped body to function as a support, and examples thereof include, but are not limited to, resins (polystyrene, polyethylene (e.g., linear low-density polyethylene (LLDPE), very-low-density/ultra-low-density polyethylene (VLDPE/ULDPE), low-density polyethylene (LDPE), or any combination of them), polypropylene, polyester, polyacrylonitrile, styrene-butadiene copolymer, (meth)acrylate polymer, fluororesin), silica gel, polysaccharides such as crosslinked dextran, polysaccharide, and agarose, glass, metals, magnetic substances, and combinations of them. The shape of the substrate may be any one that can immobilize the needle-shaped body without limitation, and may be, for example, any shape of a film or membrane, a plate, a tray, a particle (bead), a sphere, a container (a test tube, a tube, a microplate, a microtube, a cell, a cuvette, a dish, a flask, a bag), fiber, or gel. Specific examples of the substrate include, but are not limited to, a polyethylene film, a polyethylene plate, and a magnetic bead.

Immobilization of the needle-shaped body on a surface of the substrate can be performed by any means that allows binding between them to be maintained even after inserting a needle-shaped part of the needle-shaped body into a cell and evulsing the needle-shaped part. Immobilization of the needle-shaped body on a surface of the substrate can be performed, for example, through a physical adsorption method, a covalent bonding method, an ionic bonding method, embedding in the substrate, or use of a binder (adhesive), though the manner of immobilization is not limited thereto.

Any binder that can immobilize the needle-shaped body on a surface of the substrate is permitted as the "binder" applicable in the present invention, and examples thereof include, but are not limited to, polyvinyl alcohol (PVA), poly(2-hydroxyethyl methacrylate) (Poly HEMA), polyvinylpyrrolidone, vinyl acetate resin, urethane resin, vinyl chloride resin, epoxy resin, vinyl chloride-vinyl acetate copolymer resin, modified silicone resin, ethyl 2-cyanoacrylate, polystyrene, chloroprene rubber, nitrile rubber, styrene-butadiene rubber, nitrocellulose, starch, dextrin, alginate, agarose, and gelatinous proteins (gelatin, elastin, fibrin), and one or a combination of them can be used. The binder is applied onto a specific region in a surface of the substrate, and the applied binder solidifies to adhere the needle-shaped body with a part of which the binder is in contact, preferably a part of which is embedded in the binder, immobilizing the needle-shaped body on the surface of the substrate. The binder is preferably one having low bindability or adsorptivity to an "intracellularly introducible factor", which will be described later in detail, and examples of such binders include PVA and Poly HEMA. With use of such a binder in immobilizing an intracellularly introducible factor on the needle-shaped body immobilized on the substrate, binding and adsorption of the intracellularly introducible factor onto a part other than the needle-shaped body (e.g., the substrate) can be prevented, and the loss of the intracellularly introducible factor because of failure in being immobilized on the needle-shaped body can be reduced. A molecule that interacts with a cell surface protein can be contained in the binder. Examples of such molecules include fibronectin, laminin, collagen, cadherin, and fragments of them, and one or a combination of them can be used. For example, such a molecule may be mixed in advance with a binder component and then applied together onto the substrate, or applied onto to the binder on the substrate; thus, the molecule can be contained in the binder in a simple manner. With use of such a molecule in processing cells, the deterioration of the state of cells on account of change from a normal culture environment can be prevented.

In another mode, immobilization of the needle-shaped body on a surface of the substrate can be achieved through production by integral molding or integral processing of the substrate and the needle-shaped body (or needle-shaped parts). This can be performed by means of one or more methods selected from the group consisting of photolithography, dry etching, wet etching, and any combination of them according to conventionally known methods (e.g., Japanese Patent Laid-Open No. 2006-246731, Japanese Patent Laid-Open No. 2013-183706, Japanese Patent Laid-Open No. 2006-166884).

A fine uneven structure capable of accommodating a cell together with the immobilized needle-shaped body may be formed in a surface of the substrate. The fine uneven structure accommodates cells and holds each cell with a side wall (e.g., an inner wall of a concave part) and a bottom to limit the lateral movement of each cell (e.g., the horizontal movement to the surface of the substrate), thereby being capable of reducing or preventing significant damage (e.g., splitting, tearing) that may be caused by the needle-shaped body through the lateral movement of a cell. In the present invention, the "fine uneven structure" may have any shape that can exert the effects without limitation, and has a shape with recessions (concave parts) each being, for example, a dome-like, mortar-like (conical, pyramidal), or tubular (cylindrical, prismatic) shape. On the bottoms of the concave parts, the needle-shaped body is arranged and immobilized. The size of each concave part may be any size that can exert the effects without limitation, and the diameter or diagonal length and depth of each opening can be, for example, about 1 to 500 µm and 1 to 500 µm, respectively. Each concave part may accommodate one cell, or a plurality of cells.

Herein, the needle-shaped body (needle-shaped particle) in an "immobilized form on the substrate" is occasionally referred to as the "cell processing composite substrate".

In the present invention, an "intracellularly introducible factor" is immobilized on the needle-shaped body.

In an embodiment of the present invention, the "intracellularly introducible factor" is, for example, a binding substance capable of binding to an intracellular molecule. The "binding substance capable of binding to an intracellular molecule" can be any substance capable of forming a complex by binding, preferably selectively binding, more preferably specifically binding to a target intracellular molecule in a cell into which the binding substance has been introduced, and a suitable substance for a target intracellular molecule can be appropriately selected. Examples of such binding substances include, but are not limited to, nucleic acids (such as DNA, RNA, DNA-RNA hybrids), proteins (such as antibodies, antigens, enzymes, substrates, coenzymes, ligands, receptors, subunits of complexes, and fragments thereof), peptides, and low-molecular-weight compounds (e.g., Y-27632, Z-VAD-FMK). In the present invention, it is preferable that the "binding substance capable of binding to an intracellular molecule" be a substance that in itself does not directly affect expression of any gene. Here, the phrase "does not directly affect" means that introduction of the binding substance does not cause direct activation of any signal transduction pathway in cells. Preferably, the "binding substance capable of binding to an intracellular molecule" in the present invention is an antibody capable of binding, preferably selectively binding, more preferably specifically binding to a target intracellular molecule, or a fragment of the antibody. Examples of "fragments" of an antibody include Fab, Fab', F(ab')₂, Fv, scFv, dsFv, diabodies, and sc(Fv)₂, and multimers of these fragments (e.g., dimers, trimers, tetramers, polymers) can also be used in the present invention.

In another mode of the present invention, the "intracellularly introducible factor" is, for example, a factor that exerts any function or is expected to exert any function in a cell into which the factor has been introduced. Examples of such factors include bioactive substances. Examples of the "bioactive substances" include, but are not limited to, one or more substances selected from the group consisting of nucleic acids (such as DNA, RNA, DNA-RNA hybrids), plasmids, viral particles, chromosomes, and the like; proteins, amino acids, oligopeptides, polypeptides, and multi-subunit proteins; and saccharides, polysaccharides, fatty acids, cholesterols, lipids, signal transducers, ligand substances, hormonal substances, cytokines, ions, metal particles, magnetic microparticles, inorganic compounds, quantum dots, organic compounds, drugs, and so on.

In the present invention, a suitable method for the type of the "intracellularly introducible factor" to be used can be appropriately selected for binding (immobilizing) the "needle-shaped body" and the "intracellularly introducible factor" together. In the case that the "intracellularly introducible factor" to be used is the "binding substance capable of binding to an intracellular molecule" described above, binding (immobilizing) the needle-shaped body and the binding substance together needs to be performed to give strength such that the bond is maintained even in insertion of a needle-shaped part into a cell and subsequent withdrawal of the needle-shaped part from the cell, and can be performed by a method well known to those skilled in the art. Examples of the binding method include a physical adsorption method, a covalent bonding method, and an ionic bonding method, and preferred is the covalent bonding method. In the case that the binding substance is a protein or a peptide, for example, the covalent bonding method can be performed to chemically react a functional group on the surface of each needle-shaped part (e.g., a hydroxy group, an amino group, an N-hydroxysuccinimidyl group, a sulfhydryl group, an epoxy group, a vinyl group) and the carboxy terminus of the protein or peptide to form an ester bond, an amide bond, or the like. As necessary, the surface of each needle-shaped part can be treated to introduce a functional group, and this can be performed, for example, by using polylysine, polyethyleneiminevinyltrialkoxysilane, 3-mercaptopropyltrialkoxysilane, aminoalkyltrialkoxysilane, or epoxy-containing alkyltrialkoxysilane.

In binding (immobilizing) the needle-shaped particle and the binding substance capable of binding to an intracellular molecule together, they may be directly bound together, or indirectly bound via a linker.

In the case that the "intracellularly introducible factor" to be used is the "bioactive substance" described above, it is preferable that the bond (immobilization) between the factor and a needle-shaped part be maintained at least until insertion of the needle-shaped part into a cell, and the bond be separated/cleaved in the cell to allow the factor to be released into the cell. Examples of such binding modes include electrostatic bonds; bonds formed through hydrophobic interaction; chelating bonds; covalent bonds that can be cleaved in a cell such as a disulfide bond (S-S bond); bonds via a photocleavable linker; and bonds via a linker having a sequence recognizable for an enzyme such as esterase (enzymatically cleavable linker) (not limited thereto), and such binding can be performed by a method well known to those skilled in the art (Japanese Patent Laid-Open No. 2006-166884).

Identical intracellularly introducible factors may be immobilized on one needle-shaped body; otherwise, different intracellularly introducible factors may be immobilized on one needle-shaped body. Identical intracellularly introducible factors may be immobilized on a plurality of needle-shaped bodies; otherwise, different intracellularly introducible factors may be immobilized on different needle-shaped bodies.

In the present invention, a specific intracellularly introducible factor may have been immobilized in advance on the needle-shaped body; otherwise, an intracellularly introducible factor desired may be immobilized on the needle-shaped body as described above immediately before use (i.e., the needle-shaped body when provided does not need to have a specific intracellularly introducible factor already immobilized thereon).

The needle-shaped body having an intracellularly introducible factor immobilized thereon can be used for controlling intracellular reaction, and can be used in a method for producing a modified cell with intracellular reaction controlled. Hereinafter, a/the needle-shaped body is regarded as a plurality of needle-shaped bodies, unless otherwise stated.

The present production method includes:
bringing a needle-shaped body having an intracellularly introducible factor immobilized thereon into contact with a cell having a weight bound thereto;
inserting thereby a part of the needle-shaped body into the cell to introduce the intracellularly introducible factor into the cell; and then
withdrawing the part of the needle-shaped body from the cell.

Cells to be targeted in the present invention may be any of adherent cells and suspension cells without limitation, and examples of the type include, but are not limited to, pluripotent stem cells or differentiation-induced cells therefrom, hematopoietic stem cells, mesenchymal stem cells, neural stem cells, lymphocytes, neurons, glial cells, ganglion cells, pancreatic islet cells, adrenomedullary cells, myocardial cells, hepatocytes, fibroblasts, epithelial cells, endothelial cells, myoblasts, retinal epithelial cells, corneal stem cells, osteoblasts, osteoclasts, and hepatocytes. Cells of any origin are applicable without limitation, and cells of a mammal such as a human, a mouse, a rat, a monkey, a dog, a pig, a bovine, a guinea pig, and a hamster can be preferably used.

In the present invention, the term "pluripotent stem cells" refers to embryonic stem cells (ES cells) and cells potentially having pluripotency in differentiation equivalent to that of ES cells, that is, an ability to differentiate into various tissues (all of the endoderm, the mesoderm, and the ectoderm) in the living body. Examples of the cells having pluripotency in differentiation equivalent to that of ES cells include "induced pluripotent stem cells" (occasionally referred to as "iPS cells"). In the present invention, pluripotent stem cells are preferably human pluripotent stem cells. The term "induced pluripotent stem cells" refers to cells obtained through reprogramming by introducing specific factors (nuclear reprogramming factors) including Oct3/4, Sox2, Klf4, and c-Myc into mammalian somatic cells or undifferentiated stem cells. The aforementioned term "differentiation-induced cells" from pluripotent stem cells refers to cells characterized by a specific phenotype or marker expression resulting from induction of differentiation of pluripotent stem cells. The term "marker" refers to a cell antigen that is specifically expressed in a specific cell type or a gene therefor, such as a "marker protein" and a "marker gene".

In the present invention, cells collected from a living body or cultured cells may be used, and cells obtained by thawing a frozen product of any of those cells may also be used. Preferred is use of dissociated or dispersed cells.

In the present mode, a weight is bound to each cell. The cell becomes heavier by binding a weight thereto and increased impact is generated on contact between the cell and the needle-shaped body, allowing a needle-shaped part of the needle-shaped body to be inserted into the cell with ease (i.e., resulting in higher insertion probability). One or more selected from the group consisting of resins, metals, magnetic substances, and any combination of them can be used as a "weight". The form of each weight is not limited, and each weight can be, for example, in a bead-like form. In the present invention, for example, magnetic beads such as Dynabeads (registered trademark, VERITAS Corporation) can be preferably used as a "weight" for a cell. Each cell and a weight can be bound together by a method well known to those skilled in the art, and, for example, one or more of an antibody or a fragment thereof, avidin/biotin, and streptavidin/biotin can be used for binding.

Insertion of a needle-shaped part of the needle-shaped body into a cell can be performed by bringing the cell and the needle-shaped body into contact with each other. The contact between a cell and the needle-shaped body can be achieved by adding the needle-shaped body (in the form of the needle-shaped particle) to a cell (or by adding a cell to the needle-shaped body), or by adding a cell to the needle-shaped body in an immobilized form on a substrate (cell processing composite substrate). External force may be applied for bringing a cell and the needle-shaped body into contact with each other, as necessary. Here, the term "external force" refers to force applied to a cell and/or the needle-shaped body, examples thereof include centrifugal force, magnetic force, electrostatic interaction, and water flowing (water pressure), and one or more selected from them can be used. Centrifugation is needed to be under conditions that do not damage cells, and can be performed, for example, at 100 to 30000 g for 30 to 600 seconds. Water flowing (water pressure) can be generated by a pipetting operation. With use of external force, increased impact is generated on contact between a cell and the needle-shaped body, allowing a needle-shaped part of the needle-shaped body to be inserted into the cell with ease (i.e., resulting in higher insertion probability).

Insertion of a needle-shaped part of the needle-shaped particle into a cell is suitably performed in culture medium, preferably under a cell culture environment, and can be performed, for example, while culture conditions of 37°C and 5% CO₂ are maintained. In the case of short insertion time, insertion can be performed even at room temperature under the atmospheric environment, for example.

If the intracellularly introducible factor immobilized on the needle-shaped body is the "binding substance capable of binding to an intracellular molecule", insertion of a needle-shaped part of the needle-shaped body into a cell is needed to be maintained for a sufficient time that allows binding between the binding substance immobilized on the needle-shaped body inserted into the cell and an intracellular molecule of interest in the cell, and the time therefor can be appropriately set by those skilled in the art depending on the type of the binding substance or the intracellular molecule of interest, and can be, for example, 1 to 120 minutes, preferably 5 to 60 minutes, and more preferably about 30 minutes.

Thereafter, the intracellular molecule of interest can be reduced in amount or eliminated in the cell by withdrawing the needle-shaped part together with the intracellular molecule of interest bound to the binding substance, and thus a modified cell with the function of the intracellular molecule removed or reduced can be obtained.

If the intracellularly introducible factor immobilized on the needle-shaped body is the "bioactive substance", insertion of a needle-shaped part of the needle-shaped body into a cell is needed to be maintained for a sufficient time that allows the bioactive substance immobilized on the needle-shaped body to be released from the needle-shaped body in the cell (cleavage treatment is performed for the bond, as necessary), and the time therefor can be appropriately set by those skilled in the art depending on the type of the bioactive substance or the like or the bond, and can be, for example, 1 to 120 minutes, preferably 5 to 60 minutes, and more preferably about 30 minutes.

Thereafter, the bioactive substance can be increased in amount in the cell by withdrawing the needle-shaped part from the cell to allow the bioactive substance to remain in the cell, and thus a modified cell with the function of the bioactive substance introduced or enhanced can be obtained.

Withdrawal of a needle-shaped part from a cell can be performed by applying external force to pull apart the cell and the needle-shaped body in contact. Here, for the "external force", those defined above can be used.

In the present method, the steps of inserting a needle-shaped part of the needle-shaped body into a cell and withdrawing the needle-shaped part from the cell can be performed once or multiple times for the same cell. Here, the term "multiple times" means twice or more, three times or more, four times or more, or five times or more, and the upper limit is not limited but is preferably 15 times or less or 10 times or less for reducing burdens on the cell and keeping the survival state and physiological state favorable. Here, the phrase "performed multiple times" means not only the case that the steps are performed multiple times in a consecutive manner, but also the case that the steps are performed multiple times at certain time intervals.

A wide variety of modified cells are obtainable depending on the function of an intracellularly introducible factor that has been introduced. If the intracellularly introducible factor that has been introduced is the "binding substance capable of binding to an intracellular molecule" and, for example, the intracellular molecule that binds to the binding substance is a factor having a function to suppress or inhibit the activation of a cell (e.g., a factor in T cells such as CIN85, Sts-2, and Cbl (Mei Suen Kong et al., Science Signaling 05 Feb 2019: Vol. 12, Issue 567, eaav4373)), removing or reducing the function of the factor gives a modified cell with the intracellular reaction of the factor controlled, specifically, an activated modified cell. If the intracellular molecule that binds to the binding substance is a factor having a function to suppress or inhibit the induction of differentiation of a cell, removing or reducing the function of the factor gives a modified cell with the intracellular reaction of the factor controlled, specifically, a modified cell induced to differentiate (examples of modified cells are not limited to those).

If the intracellularly introducible factor that has been introduced is the "bioactive substance", and, for example, it is a factor having a function to promote the activation of a cell, introducing or enhancing the function of the factor gives a modified cell with the intracellular reaction of the factor controlled, specifically, a modified cell with the activation promoted. If the bioactive substance is a factor having a function to promote the induction of differentiation of a cell, for example, introducing or enhancing the function of the factor gives a modified cell with the intracellular reaction of the factor controlled, specifically, a modified cell induced to differentiate (examples of modified cells are not limited to those).

The modified cells obtained can be used for analyzing the function of the intracellularly introducible factor that has been introduced.

Analysis of the function of the intracellularly introducible factor that has been introduced can be performed by analyzing the state of modified cells obtained by the method. Examples of the state of the modified cells to be analyzed include the growth rate, viability, metabolism, reactive oxygen species, and gene expression in the cells (not limited thereto), and one or more states selected from them are quantified and analyzed.

For example, if the intracellularly introducible factor that has been introduced is the "binding substance capable of binding to an intracellular molecule" and the growth rate, viability, metabolism, reactive oxygen species, or gene expression in modified cells obtained is lowered, suppressed, or lost as compared with unmodified cells, the binding substance can be determined to have such a function, in other words, the intracellular molecule targeted by the binding substance can be determined to have a function to contribute to the maintenance or promotion of the growth rate, viability, metabolism, reactive oxygen species, or gene expression. Otherwise, if the growth rate, viability, metabolism, reactive oxygen species, or gene expression in modified cells obtained is increased or induced as compared with unmodified cells, for example, the binding substance can be determined to have such a function, in other words, the intracellular molecule targeted by the binding substance has a function to suppress or inhibit the growth rate, viability, metabolism, reactive oxygen species, or gene expression.

If the intracellularly introducible factor that has been introduced is the "bioactive substance" and the growth rate, viability, metabolism, reactive oxygen species, or gene expression in modified cells obtained is lowered, suppressed, or lost as compared with unmodified cells, the bioactive substance can be determined to have a function to contribute to the lowering, suppression, or loss of the growth rate, viability, metabolism, reactive oxygen species, or gene expression in cells. Otherwise, if the growth rate, viability, metabolism, reactive oxygen species, or gene expression in modified cells obtained is increased or induced as compared with unmodified cells, for example, the bioactive substance can be determined to have a function to increase or induce the growth rate, viability, metabolism, reactive oxygen species, or gene expression.

Genes to be analyzed and proteins encoded thereby to be used in analysis of gene expression in modified cells can be any ones, but are preferably ones differing from the intracellular molecule of interest, the binding substance, and the bioactive substance to be introduced so that the behavior of cells can be clarified.

The present invention relates to, in another embodiment of the present invention, a needle-shaped particle having a binding substance capable of binding to an intracellular molecule immobilized thereon, and a cell processing composite substrate including the needle-shaped particle immobilized on a substrate. In the present embodiment, the "needle-shaped particle", the "binding substance capable of binding to an intracellular molecule", and the "cell processing composite substrate", and "immobilization" methods therefor are those shown above and according to those definitions. In the present embodiment, the "needle-shaped particle" and the "cell processing composite substrate" may be provided in a form such that the specific binding substance has been immobilized in advance on the needle-shaped particle, and, alternatively, the binding substance desired may be immobilized on the needle-shaped particle as described above immediately before use (i.e., the needle-shaped particle when provided does not need to be already immobilized on the specific binding substance).

In the present embodiment, the needle-shaped particle having a binding substance capable of binding to an intracellular molecule immobilized thereon, and the cell processing composite substrate including a needle-shaped particle having a binding substance capable of binding to an intracellular molecule immobilized thereon can be used in a method for producing a modified cell. Hereinafter, a/the needle-shaped particle is regarded as a plurality of needle-shaped particles in all cases, unless otherwise stated.

The present production method includes the following steps of:
inserting a needle-shaped part of the needle-shaped particle having a binding substance capable of binding to an intracellular molecule immobilized thereon or a needle-shaped part of a needle-shaped particle in the cell processing composite substrate including a needle-shaped particle having a binding substance capable of binding to an intracellular molecule immobilized thereon into a cell;
allowing an intracellular molecule of interest and the binding substance immobilized on the needle-shaped particle to bind together in the cell; and then
withdrawing the needle-shaped part together with the intracellular molecule bound thereto via the binding substance from the cell.

In the present embodiment, the "cell", the "inserting" a needle-shaped part of a needle-shaped particle into a cell, and the "withdrawing" the needle-shaped part from the cell, and operations therefor are those as described above and according to those definitions. In particular, insertion of a needle-shaped part of a needle-shaped particle into a cell is needed to be maintained for a sufficient time that allows binding between the binding substance immobilized on the needle-shaped part in the cell and an intracellular molecule of interest in the cell, and the time therefor can be appropriately set by those skilled in the art depending on the type of the binding substance or the intracellular molecule of interest, and can be, for example, 1 to 120 minutes, preferably 5 to 60 minutes, and more preferably about 30 minutes. Thereafter, the intracellular molecule of interest can be reduced in amount or eliminated in the cell by withdrawing the needle-shaped part together with the intracellular molecule of interest bound to the binding substance, and thus a modified cell with the function of the intracellular molecule removed or reduced can be obtained. The present embodiment allows processing of a large number of cells with simple operations and allows control of intracellular reaction without causing a factor that has been introduced to remain in cells after the operations.

In the present embodiment, a "weight" described above may be bound to each cell as necessary. The cell becomes heavier by binding a weight thereto and increased impact is generated on contact between the cell and the needle-shaped particle, allowing a needle-shaped part of the needle-shaped particle to be inserted into the cell with ease (i.e., resulting in higher insertion probability).

In the present embodiment, modified cells to be obtained in the case that the intracellularly introducible factor that has been introduced is a "binding substance capable of binding to an intracellular molecule" are those as described above and according to that definition. The modified cells obtained can be used for analyzing the function of the intracellular molecule of interest. In the present embodiment, analysis of the function of the intracellular molecule of interest can be performed by analyzing the state of modified cells obtained by the method with the function of the intracellular molecule removed or reduced. Examples of the state of the modified cells to be analyzed include the growth rate, viability, metabolism, reactive oxygen species, and gene expression in the cells (not limited thereto), and determination can be made as described above by quantifying and analyzing one or more selected from them.

Hereinafter, the present invention will be described in more detail with reference to Examples, but the present invention is not limited to those Examples.

### Examples

### Experiment 1: Modification of Cells by Processing with Cell Processing Composite Substrate

### 1-1. Production of cell processing composite substrate

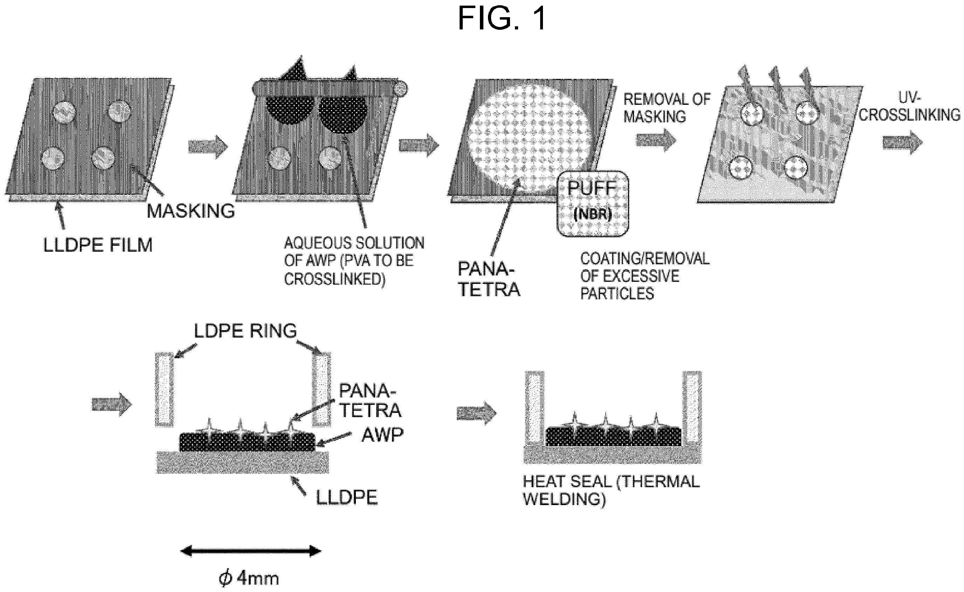

Figure 1 shows the summary of a method for producing a cell processing composite substrate having needle-shaped particles immobilized thereon in the present experiment. A film made of linear low-density polyethylene (LLDPE) was covered with a film made of LLDPE with holes of ϕ 4 mm (masking), 1% aqueous solution of water-soluble photosensitive resin (BIOSURFINE (R) AWP (manufactured by Toyo Gosei Co., Ltd.)) was added, and excessive portions of the solution were then swept off with a cylinder made of polystyrene.

Needle-shaped particles (Pana-Tetra (R) WZ-0501 (manufactured by AMTEC Co., Ltd.)) were attached onto a sponge puff (107 for emulsion pact (NBR) (manufactured by Shiseido Company, Limited)), and by using the resultant the surface of the substrate to which the photosensitive resin had been added was lightly tapped multiple times to perform coating with the Pana-Tetra.

The masking was peeled off, and the photosensitive resin was UV-crosslinked by means of a low-pressure mercury lamp (TUV15W/G15T8 (manufactured by Koninklijke Philips N. V.)) to adhere the Pana-Tetra. The UV irradiation was carried out from a distance of 12 cm for 30 minutes. Subsequently, the resultant was washed with water to wash away excess portions of the Pana-Tetra with insufficient adhesion.

A ring-shaped member (inner diameter: 4 mm) made of LDPE was overlapped on each part coated with the Pana-Tetra (ϕ 4 mm) on the substrate with their centers aligned, and the resultant was heat-sealed to give a container-like cell processing composite substrate.

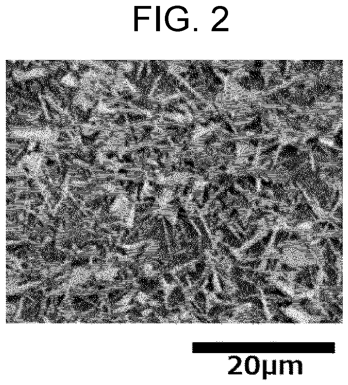

A part of the cell processing composite substrate obtained was taken for observation, and the bottom was cut out and observed through an ultra-depth multiangle microscope (VHX-D500 (manufactured by KEYENCE CORPORATION)); the observation found a coating of the Pana-Tetra in the form of needle-shaped particles immobilized in such a manner that the needle-shaped particles were partially embedded in the water-soluble photosensitive resin (Figure 2).

### 1-2. Coating of cell processing composite substrate with antibody

Into the cell processing composite substrate obtained in section 1-1., 50 µL of an anti-Cbl antibody (manufactured by BioLegend, Inc.) at a concentration of 10 µg/mL was placed, and left to stand at 37°C for 2 hours to coat the Pana-Tetra with the antibody. The resulting antibody-coated cell processing composite substrate was washed twice with 60 µL of PBS (manufactured by NACALAI TESQUE, INC.), and then used for cell processing shown below.

### 1-3. Preparation of cells

PBMCs (peripheral blood mononuclear cells, manufactured by HameCare) were thawed and cultured for 1 day. The medium used was ALyS505N-7 (manufactured by Cell Science & Technology Institute, Inc.) supplemented with 10% fetal bovine serum (manufactured by Thermo Fisher Scientific).

To promote the activation of the PBMCs, Dynabeads Human T-Activator CD3/CD28 (manufactured by Thermo Fisher Scientific) in the same number as the number of the cells were added to the cell culture, and reacted at 4°C for 1 hour to stimulate the PBMCs and bind the beads (weights) to the surfaces of the cells.

### 1-4. Processing of cells with cell processing composite substrate and evaluation

To the antibody-coated cell processing composite substrate obtained in section 1-2., 2.5 × 10⁵ cell of the PBMCs stimulated in section 1-3. were added together with the medium, and through centrifugation (21880 g, 23°C, 3 minutes) the needle-shaped parts of the Pana-Tetra coated with the anti-Cbl antibody were inserted into the cells, which were left to stand as they were at 23°C for 30 minutes.

The cells were lifted and peeled off from the cell processing composite substrate by a pipetting operation, collected in a 96-well plate with addition of 150 µL of medium, and cultured at 37°C in a 5% CO₂ environment for 1 day or 3 days (Example 1).

Used as Comparative Example were cells processed in the same manner as described above with use of a cell processing composite substrate prepared in the same manner as for the above cell processing composite substrate except that neither an anti-Cbl antibody nor Pana-Tetra was applied for coating, or a cell processing composite substrate prepared in the same manner as for the above cell processing composite substrate except that Pana-Tetra was not coated with an anti-Cbl antibody (the cells processed with the former cell processing composite substrate are referred to as "Comparative Example 1", and the cells processed with the latter cell processing composite substrate are referred to as "Comparative Example 2").

One day and three days after the initiation of culture with a 96-well plate, the cell cultures were collected and stained with an anti-CD3 antibody (fluorescence: APC-Cy7, manufactured by BioLegend, Inc.), an anti-CD25 antibody (fluorescence: APC, manufactured by BioLegend, Inc.), and an anti-CD137 antibody (fluorescence: Brilliant Violet 421, manufactured by BioLegend, Inc.), and the staining intensities of the antibodies were then analyzed with the flow cytometer CytoFLES S (manufactured by Beckman Coulter, Inc.). In the analysis, CD3-positive populations were sorted out, and CD25- and/or CD137-positive rates were calculated from them. As a reference for determination of positiveness and negativeness, non-stained cells were used.

### 1-5. Results

Generally, PBMCs are known to be activated through intracellular signal cascade based on CD3 stimulation and undergo the promotion of expression of the activation markers CD25 and CD137 (Endocrine Journal 2005, 52(5), 635-641; J Immunol Methods. 2008 Nov 30; 339(1): 23-37.). On the other hand, it is known that part of the intracellular signal cascade based on CD3 stimulation is inhibited by the ubiquitin ligase Cbl and the activation of the cells is suppressed, that is, the expression of CD25 and CD137 is suppressed (Science Signaling 05 Feb 2019: Vol. 12, Issue 567, eaav4373).

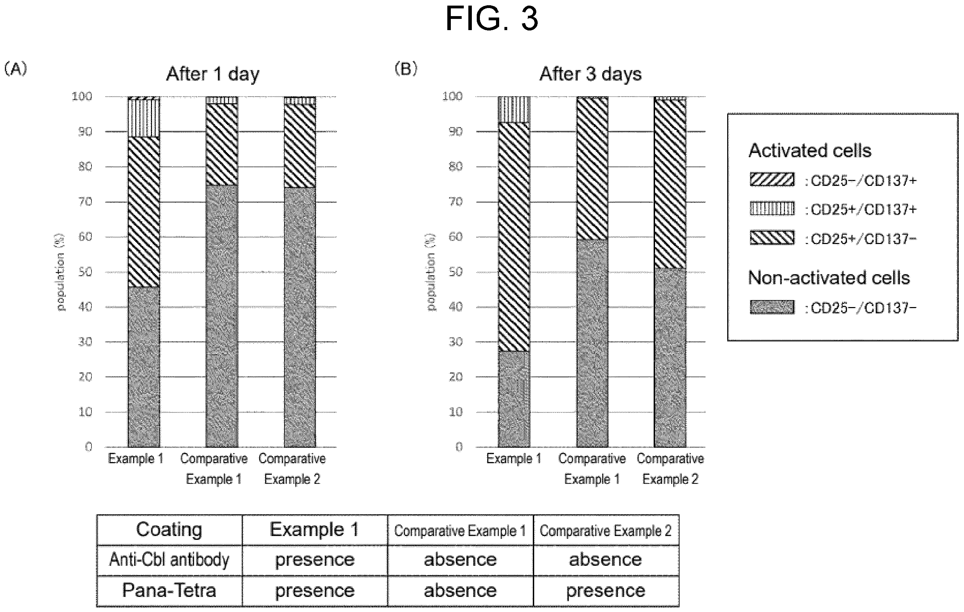

It was found for the cells subjected to insertion and evulsion of the needle-shaped parts of Pana-Tetra coated with an anti-Cbl antibody (Example 1) that the proportion of non-activated cells (CD25⁻ and CD137⁻) was lower and the proportion of activated cells indicated by a phenotype of CD25⁺ and CD137-, that indicated by a phenotype of CD25⁺ and CD137⁺, and that indicated by a phenotype of CD25⁺ and CD137- were largely higher than those of Comparative Example 1 and Comparative Example 2 (Figure 3). In addition, the proportion of activated cells after 3 days of culture was higher than that after 1 day of culture in all of Example 1, Comparative Example 1, and Comparative Example 2, and the proportion of activated cells in Example 1 was higher than those in Comparative Example 1 and Comparative Example 2; these results confirmed that the progression of activation in Example 1 was accelerated as compared with Comparative Example 1 and Comparative Example 2 and the intensity was also higher (Figure 3(A) vs. Figure 3(B)).

These results suggest that when the needle-shaped parts of Pana-Tetra coated with an anti-Cbl antibody, which had been inserted into PBMCs and left inside to allow the anti-Cbl antibody on the needle-shaped parts to bind to Cbl in the cells, were evulsed, Cbl bound to the anti-Cbl antibody was withdrawn together from the cells, and hence the inhibition by Cbl in the intracellular signal cascade based on CD3 stimulation was avoided and the activation of the cells was promoted. The influence of removal of Cbl by the processing was confirmed to possibly provide a long-term effect over at least 3 days, not a temporary effect.

Experiment 2: Increasing Efficiency of Processing with Cell Processing Composite Substrate by Binding Weight to Each Cell

### 2-1. Preparation of cells and binding of weight

Jurkat E6.1 (purchased from DS Pharma Biomedical Co., Ltd.) was cultured with ALyS505N-0 (manufactured by Cell Science & Technology Institute, Inc.) supplemented with 2% fetal bovine serum (manufactured by Thermo Fisher Scientific).

Dynabeads Human T-Activator CD3/CD28 (manufactured by Thermo Fisher Scientific) in the same number as the number of the cells were added to the cell culture, and reacted at 4°C for 1 hour to bind the beads as weights to the surfaces of the Jurkat.

### 2-2. Efficiency of insertion of Pana-Tetra needle parts by centrifugation of cells

To the cell processing composite substrate obtained in section 1-1., 2.5 × 10⁵ cells of the weight-bound Jurkat prepared in section 2-1., were added together with the medium (50 µL). This was centrifuged (2000 g, 23°C, 1 minute) to insert the needle-shaped parts of the Pana-Tetra into the cells, which were peeled off by pipetting, and again centrifuged; these operations were repeated. Centrifugation was performed 10 times in total.

After the last (10th) centrifugation, the cells were suspended by pipetting, and mixed with trypan blue solution (manufactured by FUJIFILM Wako Pure Chemical Corporation) in the equal volume. The resultant was set in a hemocytometer and observed through a microscope, stained cells and unstained cells were counted as dead cells and viable cells, respectively, and the viable cell rate was calculated from the values (Example E).

Used as Comparative Example was the viability of cells processed in the same manner as for Example E except that a cell processing composite substrate prepared in the same manner as for the above cell processing composite substrate except that Pana-Tetra was not coated was used (Comparative Example F).

Additionally used were the viability of cells processed in the same manner as for Example E except that Jurkat not bound to a weight was used (Comparative Example C), and the viability of cells processed in the same manner as for Comparative Example F except that Jurkat not bound to a weight was used (Comparative Example D).

Furthermore used were the viability of cells processed in the same manner as for Comparative Example C except that the number of cells to be added was quadrupled (Reference Example A), and the viability of cells processed in the same manner as for Comparative Example D except that the number of cells to be added was quadrupled (Reference Example B).

### 2-3. Results

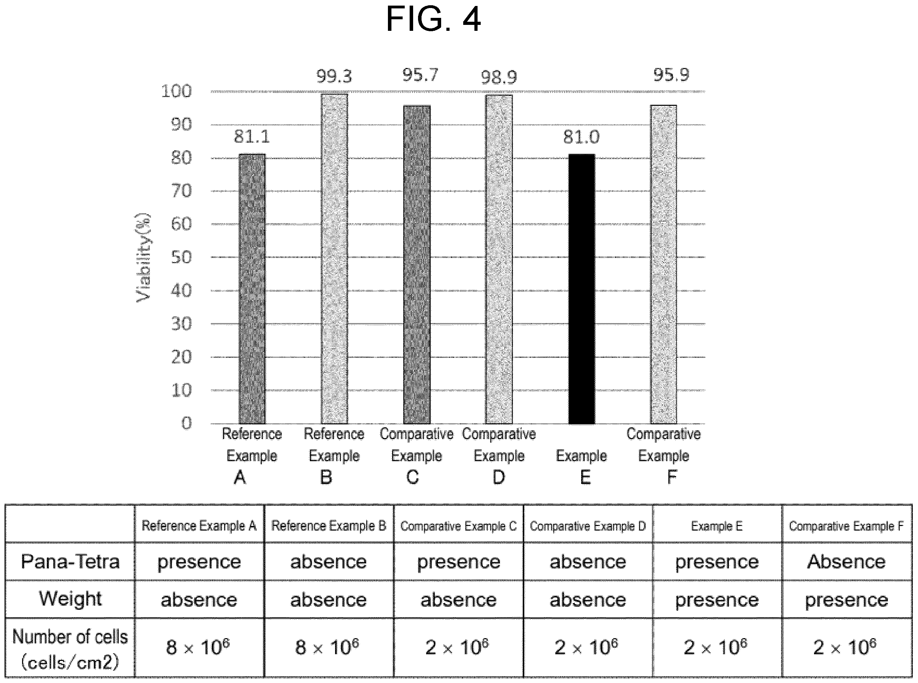

Figure 4 shows the viabilities of the cells after repeated centrifugation.

The cell viability of cells having the weights bound thereto after being allowed to act on the cell processing composite substrate by centrifugation (Example E, 81%) was confirmed to be lower than the viability of the cells after being allowed to act on a cell processing composite substrate not coated with Pana-Tetra (Comparative Example F, 95.9%). This reduction in viability can be said to be caused by repeated insertion of the needle-shaped parts of Pana-Tetra into cells through repeated centrifugation. That is, the reduction in viability indicates the success in inserting the needle-shaped parts of Pana-Tetra into cells, and it follows that the lower the viability, the higher the efficiency of insertion of the needle-shaped parts of Pana-Tetra into cells.

On the other hand, the cell viability of cells not having a weight bound thereto after being allowed to act on the cell processing composite substrate by centrifugation (Comparative Example C, 95.7%) was lower than the viability of the cells after being allowed to act on a cell processing composite substrate not coated with Pana-Tetra (Comparative Example D, 98.9%), but higher than the viability when cells having the weights bound thereto were used (Example E, 81%). This result suggests that the weight attached to each cell increased the force caused by centrifugation to press the cell to a needle-shaped part of Pana-Tetra, facilitating insertion of the needle-shaped part into the cell.

Even for cells not having weights bound thereto, it was found that increasing the number of cells (Reference Example A, 81.1%) resulted in a viability comparable to that when cells having the weights bound thereto were used. However, large numbers of cells may be unpreferable in practical use because lowered processing efficiency occasionally results.

### Experiment 3: Modification of Mesenchymal Stem Cells by Processing with Cell Processing Composite Substrate

### 1-1. Production of cell processing composite substrate

A film made of linear low-density polyethylene (LLDPE) was covered with a film made of LLDPE with holes of ϕ 4 mm (masking), 2% aqueous solution of water-soluble photosensitive resin (BIOSURFINE (R) AWP (manufactured by Toyo Gosei Co., Ltd.)) was added, and excessive portions of the solution were then swept off with a spatula made of silicone.

On the substrate, 5% ethanol dispersion of needle-shaped particles (Pana-Tetra (R) WZ-0501L (manufactured by AMTEC Co., Ltd.); average needle length: 20 µm) was dropped, and coating with the Pana-Tetra was performed by using a bar coater (No. 20) .

The masking was peeled off, and the photosensitive resin was UV-crosslinked by means of a low-pressure mercury lamp (TUV15W/G15T8 (manufactured by Koninklijke Philips N. V.)) to adhere the Pana-Tetra. The UV irradiation was carried out from a distance of 12 cm for 30 minutes. Subsequently, the resultant was washed with water to wash away excess portions of the Pana-Tetra with insufficient adhesion.

A ring-shaped member (inner diameter: 4 mm) made of LDPE was overlapped on each part coated with the Pana-Tetra (ϕ 4 mm) on the substrate with their centers aligned, and the resultant was heat-sealed to give a container-like cell processing composite substrate.

### 3-2. Coating of cell processing composite substrate with antibody

Into the cell processing composite substrate obtained in section 3-1., 25 µL of an anti-GSK3β antibody (manufactured by BioLegend, Inc.) at a concentration of 40 µg/mL was placed, and left to stand at 37°C for 3 hours to coat the Pana-Tetra with the antibody. The resulting antibody-coated cell processing composite substrate was washed once with 60 µL of PBS (manufactured by NACALAI TESQUE, INC.), and then used for cell processing shown below.

### 3-3. Preparation of cells

MSCs (mesenchymal stem cells, manufactured by PromoCell GmbH) were thawed and cultured for 15 days. The medium used was Cellartis (R) MSC Xeno-Free Culture Medium (manufactured by Takara Bio Inc.).

### 3-4. Processing of mesenchymal stem cells with cell processing composite substrate and evaluation

To the antibody-coated cell processing composite substrate obtained in section 3-2., 1.0 × 10⁵ cells of the MSCs prepared in section 3-3. were added together with the medium, and through centrifugation (2000 g, 4°C, 3 minutes) the needle-shaped parts of the Pana-Tetra coated with the anti-GSK3β antibody were inserted into the cells, which were left to stand as they were at 37°C for 30 minutes.

The cells were lifted and peeled off from the cell processing composite substrate by a pipetting operation, collected in a 48-well plate with exchanging the medium with 500 µL of mesenchymal stem cell neurodifferentiation medium, and cultured at 37°C in a 5% CO₂ environment for 3 days (Example 2).

As Comparative Example, cells processed with use of a cell processing composite substrate prepared in the same manner as for the above cell processing composite substrate except that an anti-GSK3β antibody was not applied for coating are referred to as "Comparative Example 3", cells processed in the same manner as described above with use of a cell processing composite substrate prepared in the same manner as for the above cell processing composite substrate except that neither Pana-Tetra nor an anti-GSK3β antibody was applied for coating and an anti-GSK3β antibody in an amount equivalent to 1 µg was added to the medium in centrifugation on the substrate as "Comparative Example 4", and cells processed in the same manner as described above with use of a cell processing composite substrate prepared in the same manner as for the above cell processing composite substrate except that neither Pana-Tetra nor an anti-GSK3β antibody was applied for coating as "Comparative Example 5".

Three days after the initiation of culture with a 48-well plate, the cell cultures were collected and stained with an anti-CD56 antibody (fluorescence: BV421, manufactured by BioLegend, Inc.), and the staining intensity was then analyzed with the flow cytometer CytoFLES S (manufactured by Beckman Coulter, Inc.). In the analysis, CD56-positive rates were calculated. As a reference for determination of positiveness and negativeness, cells on which an isotype antibody (fluorescence: BV421, manufactured by BioLegend, Inc.) was allowed to act under the same conditions were used.

### 3-5. Results

MSCs are known to undergo progression of differentiation into neurons and promotion of expression of the neuronal marker CD56 through the use of commercially available neurodifferentiation medium, though the detailed mechanism is still unclear. On the other hand, it is known that part of intracellular signal cascade involved in the neurodifferentiation of somatic stem cells is inhibited by GSK3β and the differentiation of the cells is suppressed, that is, the expression of CD56 is suppressed (Cell Biology International 2012 36, 967-972).

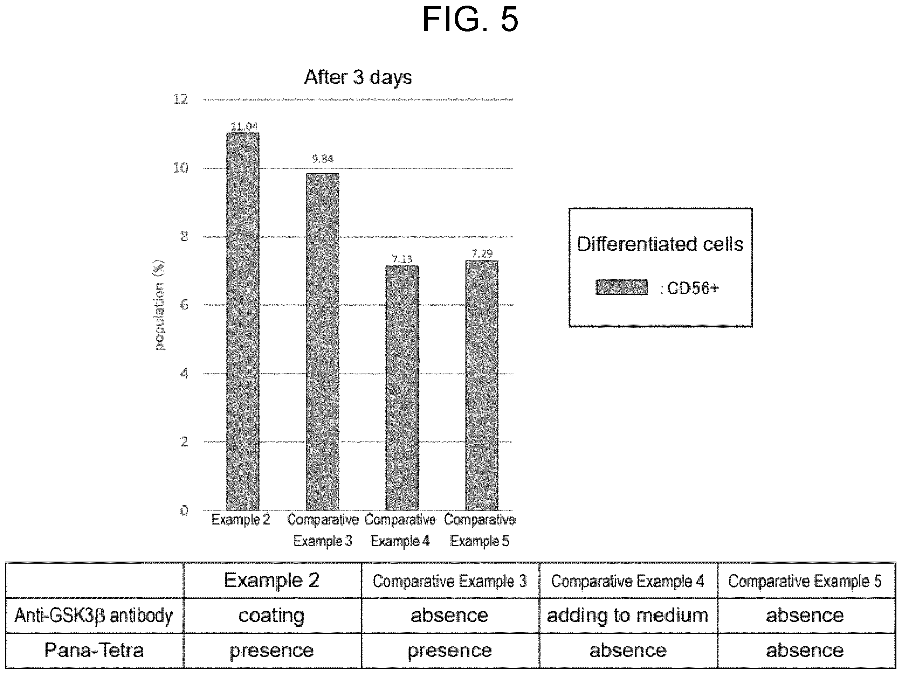

It was found for the cells subjected to insertion and evulsion of the needle-shaped parts of Pana-Tetra coated with an anti-GSK3β antibody (Example 2) that the proportion of differentiated cells indicated by a phenotype of CD56⁺ was higher than those of Comparative Example 3, Comparative Example 4, and Comparative Example 5 (Figure 5).

These results suggest that when the needle-shaped parts of Pana-Tetra coated with an anti-GSK3β antibody, which had been inserted into MSCs and left inside to allow the anti-GSK3β antibody on the needle-shaped parts to bind to GSK3β in the cells, was evulsed, GSK3β bound to the anti-GSK3β antibody was withdrawn together from the cells, and hence the inhibition by GSK3β in the intracellular signal cascade was avoided and differentiation into neurons was promoted.

The results of Experiments 1, 2, and 3 described above confirmed that an intracellularly introducible factor can be introduced into and allowed to act on a large number of cells with simple and efficient operations by bringing needle-shaped particles having the intracellularly introducible factor immobilized thereon into contact with cells each having a weight bound thereto, and the intracellular reaction can be controlled.

## Claims

1. A method for producing a modified cell, the method comprising the steps of:
bringing a needle-shaped body having an intracellularly introducible factor immobilized thereon into contact with a cell having a weight bound thereto and inserting a part of the needle-shaped body into the cell to introduce the intracellularly introducible factor into the cell; and
withdrawing the part of the needle-shaped body from the cell.

2. The method according to claim 1, wherein the weight is made of one or more substances selected from the group consisting of resins, metals, magnetic substances, and any combination of them.

3. The method according to claim 1 or 2, wherein the weight is in a bead-like form.

4. The method according to any one of claims 1 to 3, wherein the step of inserting a part of the needle-shaped body into the cell and/or the step of withdrawing the part of the needle-shaped body from the cell are/is performed by means of one or more external forces selected from the group consisting of centrifugal force, magnetic force, water flowing, water pressure, and electrostatic interaction.

5. The method according to any one of claims 1 to 4, wherein the intracellularly introducible factor is a binding substance capable of binding to an intracellular molecule, and the method comprises withdrawing the needle-shaped body from the cell to withdraw an intracellular molecule bound to the binding substance in the cell together with the needle-shaped body from the cell.

6. The method according to claim 5, wherein the binding substance is a nucleic acid, a protein, a peptide, or a low-molecular-weight compound.

7. The method according to claim 5 or 6, wherein the binding substance is an antibody or a fragment thereof.

8. The method according to any one of claims 1 to 4, wherein the intracellularly introducible factor is a bioactive substance, and the method comprises withdrawing the needle-shaped body from the cell to allow the intracellularly introducible factor to remain in the cell.

9. The method according to claim 8, wherein the bioactive substance is one or more substances selected from the group consisting of nucleic acids, peptides, proteins, saccharides, polysaccharides, fatty acids, cholesterols, lipids, signal transducers, ligand substances, hormonal substances, cytokines, ions, metal particles, magnetic microparticles, inorganic compounds, quantum dots, organic compounds, and drugs.

10. The method according to claim 8 or 9, wherein a bond between the bioactive substance and the needle-shaped body is an intracellularly separable bond.

11. The method according to claim 10, wherein the bond between the intracellularly introducible factor and the needle-shaped body is at least one selected from the group consisting of electrostatic bonds, bonds formed through hydrophobic interaction, chelating bonds, intracellularly cleavable covalent bonds, bonds via a photocleavable linker, and bonds via an enzymatically cleavable linker.

12. The method according to any one of claims 1 to 11, wherein the needle-shaped body is a needle-shaped particle.

13. The method according to claim 12, wherein a needle-shaped part of the needle-shaped particle has a length of 1 to 50 µm.

14. The method according to claim 12 or 13, wherein the needle-shaped particle is made of zinc oxide.

15. The method according to any one of claims 1 to 14, wherein the needle-shaped body is in an immobilized form on a substrate.

16. The method according to claim 15, wherein the substrate comprises a binder on a surface of the substrate, and a part of the needle-shaped body is immobilized on the substrate via the binder.

17. The method according to claim 16, wherein the binder consists of a protein-nonadsorptive material.

18. The method according to claim 15, wherein the needle-shaped body is a needle-shaped body produced by using one or more selected from the group consisting of photolithography, dry etching, wet etching, and any combination of them.

19. The method according to any one of claims 1 to 18, wherein the substrate comprises a fine uneven structure capable of accommodating a cell in a face of the substrate with the needle-shaped body.

20. A method for analyzing a function of an intracellularly introducible factor, the method comprising the step of: analyzing a state of a modified cell produced by using the method according to any one of claims 1 to 19.

21. The method according to claim 20, wherein, in the step of analyzing a state of a modified cell, analysis is performed for the cell on one or more selected from the group consisting of a growth rate, a viability, metabolism, quantification of reactive oxygen species, and gene expression.

22. A needle-shaped particle having a binding substance capable of binding to an intracellular molecule immobilized thereon.

23. The needle-shaped particle according to claim 22, wherein a needle-shaped part of the needle-shaped particle has a length of 1 to 50 µm.

24. The needle-shaped particle according to claim 22 or 23, wherein the needle-shaped particle is made of zinc oxide.

25. The needle-shaped particle according to any one of claims 22 to 24, wherein the binding substance is a nucleic acid, a protein, a peptide, or a low-molecular-weight compound.

26. The needle-shaped particle according to claim 25, wherein the binding substance is an antibody or a fragment thereof.

27. A cell processing composite substrate comprising a needle-shaped particle immobilized on a substrate.

28. The cell processing composite substrate according to claim 27, wherein a needle-shaped part of the needle-shaped particle has a length of 1 to 50 µm.

29. The cell processing composite substrate according to claim 27 or 28, wherein the needle-shaped particle is made of zinc oxide.

30. The cell processing composite substrate according to any one of claims 27 to 29, wherein the substrate consists of a resin.

31. The cell processing composite substrate according to any one of claims 27 to 30, wherein the substrate comprises a binder on a surface of the substrate, and a part of the needle-shaped particle is immobilized on the substrate via the binder.

32. The cell processing composite substrate according to claim 31, wherein the binder consists of a protein-nonadsorptive material.

33. The cell processing composite substrate according to any one of claims 27 to 32, wherein a fine uneven structure capable of accommodating a cell is formed in a face of the substrate with the needle-shaped particle immobilized thereon.

34. The cell processing composite substrate according to any one of claims 27 to 33, wherein a binding substance capable of binding to an intracellular molecule is immobilized on the needle-shaped particle.

35. The cell processing composite substrate according to claim 34, wherein the binding substance is a nucleic acid, a protein, a peptide, or a low-molecular-weight compound.

36. The cell processing composite substrate according to claim 35, wherein the binding substance is an antibody or a fragment thereof.

37. A method for producing a modified cell, wherein a function of an intracellular molecule is removed or reduced, the method comprising the steps of:
inserting a part of the needle-shaped particle according to any one of claims 22 to 26 or the needle-shaped particle in the cell processing composite substrate according to any one of claims 34 to 36 into a cell;
allowing the intracellular molecule and the binding substance immobilized on the needle-shaped particle to bind together; and
withdrawing the needle-shaped particle together with the intracellular molecule bound via the binding substance from the cell.

38. The method according to claim 37, wherein the modified cell is used for analyzing the function of the intracellular molecule.

39. The method according to claim 37 or 38, wherein the step of inserting a part of the needle-shaped particle into a cell and/or the step of withdrawing the needle-shaped particle from the cell are/is performed by means of one or more external forces selected from the group consisting of centrifugal force, magnetic force, water flowing, water pressure, and electrostatic interaction.

40. A method for analyzing a function of an intracellular molecule, the method comprising the steps of:
inserting a part of the needle-shaped particle according to any one of claims 22 to 26 or the needle-shaped particle in the cell processing composite substrate according to any one of claims 34 to 36 into a cell;
allowing the intracellular molecule and the binding substance immobilized on the needle-shaped particle to bind together;
withdrawing the needle-shaped particle together with the intracellular molecule bound thereto via the binding substance from the cell; and
analyzing a state of the cell.

41. The method according to claim 40, wherein, in the step of analyzing a state of the cell, analysis is performed for the cell on one or more selected from the group consisting of a growth rate, a viability, metabolism, quantification of reactive oxygen species, and gene expression.

42. The method according to claim 40 or 41, wherein the step of inserting a part of the needle-shaped particle into a cell and/or the step of withdrawing the needle-shaped particle from the cell are/is performed by means of one or more external forces selected from the group consisting of centrifugal force, magnetic force, water flowing, water pressure, and electrostatic interaction.
